# EUROPEAN PATENT SPECIFICATION

(11) **EP 4 486 314 B1**
(45) Date of publication and mention of the grant of the patent: **25.11.2026**
(21) Application number: 23719934.4
(22) Date of filing: 28.02.2023
(51) Int. Cl.: A61K 31/047, A61K 31/365, A61P 17/00, A61P 17/06, A61P 17/08, A61P 31/10, A61P 17/14, A61Q 19/00, A61P 17/16, A61Q 5/00, A61Q 7/00, A61K 47/69, A61K 8/49

(54) **SCLAREOLIDE FOR IMPROVING SCALP CONDITIONS AND HAIR GROWTH**
SCLAREOLIDE ZUR VERBESSERUNG DES ZUSTANDS DER KOPFHAUT UND DES HAARWACHSTUMS
SCLARÉOLIDE POUR AMÉLIORER L'ÉTAT DU CUIR CHEVELU ET LA CROISSANCE DES CHEVEUX

(30) Priority: 01.03.2022 US 202263315502 P; 22.04.2022 US 202263333959 P
(43) Date of publication of application: 08.01.2025
(73) Proprietor: ISP Investments LLC, Wilmington, DE 19805 (US)
(72) Inventor: QU, Xin, Shanghai, 201108 (CN); CAPALLERE, Christophe, 06200 Nice (FR); ARCIONI, Marianne, 06620 Le Bar Sur Loup (FR); GARCIA, Imane, 06000 Nice (FR); PERRIN, Armelle, 06560 Valbonne (FR); LE MESTR, Audrey, 06600 Antibes (FR); MEYRIGNAC, Céline, 83480 Puget sur Argens (FR); DE FEIJ, Eric-Jan, 2406JP Alphen aan den Rijn (NL)
(74) Representative: Kutzenberger Wolff & Partner
(86) International application number: PCT/US2023/014146
(87) International publication number: WO 2023/167867

(56) References cited:
- WO-A1-01/74327
- WO-A1-2016/188988
- WO-A2-02/30385
- WO-A2-99/63978
- CN-A- 112 006 970
- ANONYMOUS: "WayBackMachine - SclareanceTM Biofunctional", WAYBACKMACHINE, 3 December 2022 (2022-12-03), pages 1 - 1, Retrieved from the Internet <URL:https://web.archive.org/web/20221203182651/https://www.ashland.com/industries/personal-and-home-care/hair-care/sclareance-biofunctional>
- DATABASE GNPD [online] "MINTEL"; 10 May 2017 (2017-05-10), "Pyrithione Zinc Antidandruff Shampoo", retrieved from https://www.gnpd.com/sinatra/recordpage/4788737/ Database accession no. 4788737
- THOMBARE SMRUTI ET AL: "Control of M. furfur using Plant Essential Oil", JOURNAL OF PURE AND APPLIED MICROBIOLOGY, vol. 6, no. 4, 31 December 2012 (2012-12-31), pages 1955 - 1958, Retrieved from the Internet <URL:https://microbiologyjournal.org/control-of-m-furfur-using-plant-essential-oil/#>
- DATABASE GNPD [online] MINTEL; 10 May 2017 (2017-05-10), ANONYMOUS: "Pyrithione Zinc Antidandruff Shampoo", XP093059222, retrieved from https://www.gnpd.com/sinatra/recordpage/4788737/ Database accession no. 4788737
- SMRUTI THOMBARE ET AL: "Control of M. furfur using Plant Essential Oil", JOURNAL OF PURE AND APPLIED MICROBIOLOGY, 31 December 2012 (2012-12-31), pages 1 - 1, XP093052951, Retrieved from the Internet <URL:https://microbiologyjournal.org/control-of-m-furfur-using-plant-essential-oil/#> [retrieved on 20230608]
- ROSS NINA: "Clary Sage Essential Oil: Is It Good For Your Hair?", NINA ROSS HAIR THERAPY, 10 November 2020 (2020-11-10), pages 1 - 4, XP093059229, Retrieved from the Internet <URL:https://ninaross.co/blogs/taking-care-of-your-hair-posts/clary-sage-for-hair-is-clary-sage-good-for-your-hair> [retrieved on 20230629]
- SCHWARTZ J R ET AL: "The role of oxidative damage in poor scalp health: ramifications to causality and associated hair growth", INTERNATIONAL JOURNAL OF COSMETIC SCIENCE, KLUWER ACADEMIC PUBLISHERS, DORDRECHT, NL, vol. 37, 17 November 2015 (2015-11-17), pages 9 - 15, XP071470275, ISSN: 0142-5463, DOI: 10.1111/ICS.12289
- ANONYMOUS: "Sclareance biofunctional presentation", 23 May 2022 (2022-05-23), pages 1 - 42, XP093052925, Retrieved from the Internet <URL:https://www.azelis.com/sites/default/files/uploads/Ashland%20Documents/sclareance%20biofunctional-%20presentation_PDF.pdf> [retrieved on 20230608]
- ARCIONI M. ET AL: "713 - Salvia sclarea derivative ingredient as an alternative solution to zinc pyrithione: Innovative scalp 3D model adapted to study anti-dandruff", JOURNAL OF INVESTIGATIVE DERMATOLOGY - 1ST INTERNATIONAL SOCIETIES FOR INVESTIGATIVE DERMATOLOGY MEETING ABSTRACT SUPPLEMENT, 10 May 2023 (2023-05-10), pages S123 - S123, XP093059188, Retrieved from the Internet <URL:https://www.sciencedirect.com/science/article/pii/S0022202X23008977?via%3Dihub> [retrieved on 20230629]

## Description

### FIELD OF THE INVENTION

The present disclosure relates to the field of personal care and more particularly to a method for improving scalp condition and hair growth, which comprises applying to the scalp a composition comprising as an active agent sclareol, sclareol-like, sclareolide or sclareolide-like and a physiologically acceptable carrier.

### BACKGROUND

The scalp refers to the skin and subcutaneous tissue that cover the head.

Abnormal or irregular desquamation of the uppermost layer of the scalp can result in the formation of large, thick cell clusters called "squamae" or "dandruff".

Dandruff is a benign but aesthetically unpleasant cosmetic scalp condition affecting up to half the world's population.

Factors which promote the appearance of dandruff are made of stress, the winter period, an excess of sebum, a moisturization defect, an impairment of the barrier function of the epidermis or colonization of the skin or the hair follicles by the yeasts *Malassezia* species. However, many unknown factors remain regarding the intimate mechanism and the factors involved in the onset of dandruff.

Comparison of healthy and dandruff scalp microbiome reveals the role of commensal bacteria and fungi in scalp health and an imbalance of the microbiome (dysbiosis) is associated with the onset of dandruff (Front Cell Infect Microbiol. 2018; 8:346).

*Cutibacterium acnes* (previously named *Propionibacterium acnes*) and *Staphylococcus epidermidis* emerged as the core commensal bacterial species, where the former was associated with a healthy scalp and the latter with dandruff scalp, while commonly occurring yeasts on the scalp are *Malassezia* species (M. species).

As with *Malassezia* species, the number of staphylococci differed both between healthy and dandruff scalps, and between different areas on the same scalp. It seems that local changes could disrupt the microbiome, rather than a condition that affects the individual as a whole.

The excessive colonization of scalp by *Malassezia species* has long been considered the main cause of dandruff. This idea is supported by improvement in dandruff, accompanied by a reduction in the number of yeast cells on the scalp, following treatment with a shampoo containing an antifungal agent such as zinc pyrithione.

A total of 17 *different Malassezia* species have been identified, for example *M*. *restricta, M. globosa, M. furfur* and among them *Malassezia restricta* is the dominant species on human skin.

*Malassezia restricta* is involved in triggering the disequilibrium between the commensals *Cutibacterium acnes* and *Staphylococcus* spp. (Del Prete S. et al. Cloning, Purification, and Characterization of a β-Carbonic Anhydrase from Malassezia restricta, an Opportunistic Pathogen Involved in Dandruff and Seborrheic Dermatitis. Int J Mol Sci. 2019;20(10):2447).

Lipases secreted from *Malassezia* contribute to the development of dandruff due to the hydrolyze of sebum triglycerides and help the yeast cells take up saturated fatty acids to generate energy. The accumulation of an excess of unsaturated fatty acids, such as oleic acid, on the skin causes skin irritation in patients suffering from dandruff (Park, M.et al. Understanding the Mechanism of Action of the Anti-Dandruff Agent Zinc Pyrithione against Malassezia restricta. Sci Rep 8, 12086 (2018).

Dandruff conditions generally respond to various local or systemic treatments. However, the efficacy of these treatments is only suspensory, demands frequent use, while daily and long-term use can lead to a phenomenon of habituation, reducing their efficacy and may be associated with a rebound phenomenon.

Moreover, the aggressiveness of certain antidandruff active agents may also affect the scalp's barrier functions and lead to worsening of the dandruff condition.

A commonly used treatment of dandruff is the topical application of antifungal agents such as zinc pyrithione, piroctone olamine, and climbazole which are normally delivered from a shampoo. Antidandruff shampoos containing zinc pyrithione help to control scalp flaking, scaling and itching (DATABASE GNPD MINTEL; 10 May 2017, "Pyrithione Zinc Antidandruff Shampoo"). Their efficacy against dandruff and their ability to reduce trans-epidermal water loss are proven (Schwartz JR et al. The role of oxidative damage in poor scalp health: ramifications to causality and associated hair growth, International Journal of Cosmetic Science 2015; 37 (Suppl. 2)). Although there is no doubt that treatments such as zinc pyrithione, piroctone olamine, and climbazole are effective antifungal agents, there are disadvantages. Zinc pyrithione, which has been classified as a category 1B carcinogen, is now prohibited for use in cosmetic products by The European Commission (Regulation (EU) 2021/1902 Regulation Bulletin), while piroctone olamine and climbazole are synthetic antifungal agents with less efficacy as compared to zinc pyrithione. The use of plants essential oils, such as Salvia sclarea oil, has been shown to reduce the growth of *Malassezia furfur* when added in a shampoo formulation (Smruti Thombare et al., Control of M. furfur using Plant Essential Oil, J Pure Appl Microbio 2012; 6(4)).

There remains a need for novel natural active agents for improving scalp conditions, preventing, reducing and/or treating dandruff conditions of the scalp, which are efficient and free of side effects.

The objective of the present invention is to provide an antidandruff agent which is non-irritant to the skin and the scalp, while at the same time effectively improves scalp condition.

The anti-inflammatory activity of sclareol and sclareolide is described in the international patent application WO2002030385 A2. The anti-microbial activity of sclareol-like and sclareolide-like compounds is already described in U.S. Pat. No. 6,150,381 concluding that sclareolide and sclareol are useful to treat acne and dermatitis. In WO 2001074327 the use of sclareolide as cell differentiation enhancer is disclosed. According to this patent differentiation enhancers like sclareolide are used to stimulate the production of lipids from epidermal cells, and concurrently increase the lipid content of the barrier. The strengthening of barrier function of the skin using sclareolide is described in WO 2002060381 A2. The use of sclareolide in cosmetic formulations used to enhance the stratum corneum function is described in US 2010 247692 A1.

For all the above reasons, sclareol and sclareolide were identified as potential candidates to help maintaining healthy scalp conditions and to avoid conditions that could promote dysbiosis.

Sclareolide is endowed with a good antibacterial activity against *Staphylococcus aureus* ATCC 25923, *Pseudomonas aeruginosa* ATCC 27950, *Escherichia coli* ATCC 25922 and *Enterococcus faecalis* ATCC 29212 (Hayet E, et al. Antibacterial and cytotoxic activity of the acetone extract of the flowers of Salvia sclarea and some natural products. Pak J Pharm Sci. 2007 Apr;20(2):146-8).

The applicant has now found that the use of a composition comprising sclareolide as an active agent makes it possible to improve scalp condition and effectively treat or prevent dandruff conditions, and in particular dandruff conditions associated with an excessive proliferation of yeasts of the *Malassezia* species on the scalp. The composition "Sclareance^{™} biofunctional" by Ashland, comprising 0.12% sclareolide from clary sage, reduced dandruff, scalp sensitivity (itching) and the growth of *Malassezia* species ("Sclareance biofunctional presentation", 23 May 2022).

Another object of the present invention is to provide an agent for improving hair growth.

Vitamin D plays an important role in skin structure wherein it stimulates differentiation of keratinocytes to form the upper layers of the epidermis (Bikle D. Vitamin D Metabolism and Function in the Skin. Mol Cell Endocrinol. 2011; 347(1-2): 80-89.).

The active form of vitamin D (1,25(OH)2D3) stimulates hair growth by upregulating factors implicated in hair follicle-inductive capacity of cultured DPCs (Noriyuki Aoi et al... 1α,25-dihydroxyvitamin D3 modulates the hair-inductive capacity of dermal papilla cells: therapeutic potential for hair regeneration. Stem Cells Transl Med. 2012 Aug; 1(8):615-26).

The active form of vitamin D (1,25(OH)2D3) in the skin is also known to improve the skin barrier including keratin 10 whose level is decreased in people suffering dandruff (Kathy Kerr et al. Epidermal changes associated with symptomatic resolution of dandruff: biomarkers of scalp health. International Journal of Dermatology 2011, 50, 102-113). The enzyme CYP27B1 metabolizes the intermediary form of vitamin D (25 OH D3) into the active form of vitamin D (1,25(OH)2D3) and plays a role in hair follicle cycle. A decrease in its expression was observed in subjects suffering alopecia (Michel L et al. Study of gene expression alteration in male androgenetic alopecia: evidence of predominant molecular signalling pathways. Br J Dermatol 2017; 177:1159-1160).

The applicant has now found that the use of a composition comprising sclareolide as an active agent was able to increase the level of CYP27B1 in healthy *ex vivo* scalp skin biopsies.

### SUMMARY

The present invention relates to a composition comprising sclareolide as an active agent and a physiologically acceptable carrier, for its therapeutic use in improving the scalp condition and hair growth, wherein improving the scalp condition is chosen among at least one of decreasing scalp sensitivity (itching), eliminating or reducing dandruff and decreasing the growth of Malassezia species, and wherein the composition comprises sclareolide in a concentration ranging from 0.001% to 0.2% by weight, relative to the total weight of the composition.

In a particular embodiment Malassezia species is *Malassezia restricta* or *Malassezia furfur.*

In another aspect the present invention relates to the cosmetic use of a composition comprising sclareolide as an active agent and a physiologically acceptable carrier, for improving the scalp condition wherein improving the scalp condition is improvement of scalp hydration and wherein the composition comprises sclareolide in a concentration ranging from 0.001% to 0.2% by weight, relative to the total weight of the composition.

### BRIEF DESCRIPTION OF THE DRAWINGS

For the purpose of illustrating aspects of the present invention, there are depicted in the drawing's certain embodiments of the invention. However, the invention is not limited to the precise arrangements and instrumentalities of the embodiments depicted in the drawings. Further, if provided, like reference numerals contained in the drawings are meant to identify similar or identical elements.
**Fig. 1** Adherent scalp flaking score (ASFS) grading method
**Fig. 2** ASFS results of scalp applied with scalp care serum with 0.12% sclareolide vs. placebo (△ directional, 0.05<p<0.1; * significant, p<0.05; *** extremely significant, p<0.001)
**Fig. 3** Loose dandruff weight of scalp applied with scalp care serum with 0.12% sclareolide vs. placebo (△ directional, 0.05<p<0.1; * significant, p<0.05; *** extremely significant, p<0.001)
**Fig. 4** Scalp sensitivity (itching) study. Scalp was applied with scalp care serum with 0.12% sclareolide vs. placebo (△ directional, 0.05<p<0.1; * significant, p<0.05; *** extremely significant, p<0.001)
**Fig. 5** Scalp barrier function results (TEWL, Tewameter) of scalp applied with scalp care serum with 0.12% sclareolide vs. placebo (△ directional, 0.05<p<0.1; * significant, p<0.05; *** extremely significant, p<0.001)
**Fig. 6** Scalp sebum results of scalp applied with scalp care serum with 0.12% sclareolide at Day 0, Day 14, and Day 28 (△ directional, 0.05<p<0.1; * significant, p<0.05; *** extremely significant, p<0.001)
**Fig. 7** Scalp hydration results of scalp applied with scalp care serum with 0.12% sclareolide at Day 0, Day 14, and Day 28 (△ directional, 0.05<p<0.1; * significant, p<0.05; *** extremely significant, p<0.001)
**Fig. 8** MIC ((Minimum inhibitory concentration) determination of sclareolide on *Malassezia restricta*
**Fig.9** Solubility of Sclareolide complex in surfactant solution after 1 hour (12% Sodium laureth sulfate /2% Cocamidopropyl betaine)
**Fig.10** Differential Scanning Calorimeter (DSC) curve of sclareolide and Sclareolide complex 1 (HP- β-cyclodextrin) and 2 (γ- cyclodextrin).

### DETAILED DESCRIPTION

The references to methods of treatment in the summary and detailed description of the invention in this description are to be interpreted as references to the compounds, pharmaceutical compositions and medicaments of the present invention for use in a method for treatment of the human (or animal) body by therapy.

Detailed embodiments of the present invention are disclosed herein; however, it is to be understood that the disclosed embodiments are merely illustrative of the invention that may be embodied in various forms. Therefore, specific structural and functional details disclosed herein are not to be interpreted as limiting.

Whenever a term is identified by reference to a range, the range will be understood to explicitly disclose every element thereof. As a non-limiting example, a range of 1-10% will be understood to include 1%, 2%, 3%, 4%, 5%, 6%, 7%, 8%, 9%, and 10%, and all values between 1 and 10%.

Where two or more substituents are referred to as being "selected from" a group of enumerated alternatives, it is meant that each substituent can be any element of that group, independent of the identity of the other substituents.

As used herein, "% refers to % by weight, that is the weight percent of a component in relation to the total weight of the skin care composition (i.e., including any carriers, vehicles, solvents, fillers, or other components added before application to the skin) unless otherwise provided.

All terms used herein are intended to have their ordinary meaning unless otherwise provided. For the purposes of describing and claiming the present invention, the following terms are defined:

The compositions described and used in the present disclosure can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "Application" as used in reference to a hair care composition means to apply or spread the compositions of the present invention onto a human skin surface such as the epidermis.

"Physiologically acceptable" as used herein means, in the skin care compositions of the invention, a medium suitable for use in contact with human skin tissue without undue toxicity, incompatibility, instability, allergic response and the like, capable of being applied to the skin, integuments or lips of the face or the body of mammals or human beings.

"Effective amount" as used herein means an amount of an agent sufficient to significantly induce a positive appearance and/or feel benefit, but low enough to avoid serious side effects (i.e., to provide a reasonable benefit to risk ratio, within the scope of sound judgment of the skilled artisan).

"Improved scalp condition" as used herein is reflected by the improvement of at least one of the following scalp conditions: improvement of scalp hydration, decrease of scalp sebum, improvement of scalp pH, decrease in scalp sensitivity (itching), reinforcement of skin barrier function, decrease of adherent dandruff and loose dandruff, and decrease of growth *of Malassezia species* or *Malassezia furfur.*

"Improving dandruff condition" as used herein is reflected by the significant decrease of adherent dandruff and loose dandruff.

"Improving hair growth" is reflected by the increase of active form of vitamin D in scalp.

"Dysbiosis" as used herein means the loss of balance within the microbiome of the scalp either due to a shift in the microbiota or a change in the ecosystem of the microbiome.

Subjects to be treated by the methods of the present invention are typically human subjects, although the methods of the present invention may be useful with any suitable subjects known to those skilled in the art, and particularly mammalian subjects including, in addition to humans, horses, cows, dogs, rabbits, fowl, sheep, and the like, for veterinary purposes.

As disclosed herein, the "active agents of the present disclosure" or "sclareol and/or a derivative thereof" include sclareol, sclareol-like, sclareolide and sclareolide-like compounds. In the present invention, the active agent is sclareolide. Sclareol, sclareol-like and sclareolide-like compounds do not fall within the scope of the claimed subject-matter.

The present disclosure relates to a method for improving scalp condition and hair growth, the method comprising: applying to the scalp a composition comprising an active agent selected among sclareol, sclareol-like, sclareolide or sclareolide-like and a physiologically acceptable carrier.

In a particular embodiment the improvement of scalp condition is chosen among at least one of the following scalp conditions: improvement of scalp hydration, decrease of scalp sebum, improvement of scalp pH, decrease in scalp sensitivity (itching), reinforcement of skin barrier function, decrease or elimination of dandruff including adherent dandruff and loose dandruff, and decrease of growth of *Malassezia* species and more specifically *Malassezia restricta or Malassezia furfur;* decrease of scalp sebum, improvement of scalp pH, reinforcement of skin barrier function are not covered by the claims.

Sclareol is an important bioactive diterpene obtained from clary sage (*Salvia sclarea L.).*

Sclareol-like agents are diterpene compounds, and include, for example, 13-episclareol, ferruginol, salvipisone, aethopisome, neoclerodane, sagequinone, romulogarzone, ortho-benzoquinone, para-benzoquinone, and clariol. Other sclareol-like compounds include abietane and icetexane diterpenoids, languidulane diterpene, paryin and pimarine diterpenes, methylene quinone diterpenoids, manoyl norditerpenoids, multicaulin, salvipimarone and pimarane diterpenoid. See, for example, the types of compounds set forth in Gonzalez et al., Can. J. Chem. 67(2), 208-212(1989); Eanthorpe et al., Phytochem. 29, 2145-2148(1990); Kouzi et al., Helv. Chim. Acta. 73(8), 2157-2164 1990); Abraham, Phytochem. 36(6) 1421-1424(1994); Ulubelen et al. Phytochem. 36(4), 971-974 (1994); Hanson, Nat. Prod. Rep., 13, 59-71 (1996) and Topcu et al., J. Nat. Prod. 59, 734-737 (1996).

Sclareolide and Sclareolide-like compounds are fused-ring diterpene compounds that may be derived from sclareol by chemical or biological techniques known to those skilled in the art; and include, for example, ambrox, and wiedenol. See, for example, the types of compounds set forth in Hanson, Nat. Prod. Rep. 13, 59-71 (1996); Chackalamanni et al., Tetrahedron Letters 36, 5315-5318 (1995); Barrero et al., Tetrahedron Letters 35, 2945-2948 (1994); Martres et al. Tetrahedron Letters 34, 801-8084 (1993) and Barrero et al., Tetrahedron 49(5), 10405-10412 (1993).

The active agents described above are cosmetically or pharmaceutically acceptable analogs, derivatives, or salts of sclareol or sclareolide. In the practice of the present disclosure, the active agents may alternatively be substituted with alkyl (both unsaturated and saturated, and branched and unbranched, such as methyl, ethyl, or isopropyl), aryl, halogen, hydroxy, alkoxy, and amino groups, as will be apparent to those skilled in the art.

Any of the active agents of the present disclosure may be present as an optical isomer, or chiral compound, or as a mixture of optical isomers and chiral compounds.

Sclareol used according to the present disclosure is advantageously obtained by solvent extraction of clary sage. This diterpene is not widely distributed and the most convenient sources are flower heads of clary sage plant. Sclareol is obtained by solvent extraction of clary sage. U.S. Pat. No. 3,060,172 describes a process for the isolation of sclareol from clary sage. See also, U.S. Pat. No. 5,908,771 describes a method for regeneration of salvia species, and U.S. Pat. No. 5,945,546 describes a method for purifying sclareolide, which are incorporated herein in their entirety by reference. Alternatively, sclareol may also be obtained via genetically modified microorganisms such as found in U.S. Pat. No. 9,670,494 and 9,745,602.

Sclareolide (CAS Number 564-20-5) used according to the present disclosure is advantageously prepared by either chemical oxidation followed by lactonization of sclareol or by biotransformation of sclareol using a yeast strain. Exemplary methods of producing sclareolide include those methods disclosed in U.S. Patent Nos. 5,525,728 to Schneider et al., U.S. Patent No. 5,247,100 to Gerke et al., and German Patent DE 3942358 to Gerke et al. Briefly, these processes use a ruthenium catalyst and an oxidation step to convert sclareol into a crude sclareolide product.

Other exemplary methods of converting sclareol to sclareolide that are more commonly used include the biotransformation and fermentation methods described in U.S. Patent Nos. 4,970,163 and 5,212,078, both to Farbood et al. Sclareolide produced by these described methods is normally provided in wet or dry cake form and is generally from about 90% to 95% pure. Furthermore, sclareolide in higher purity can be obtained through crystallization of the wet or dry cake. Sclareolide has also been reported to have therapeutic properties. See, WO 96/20704 to Braquet et al.

In a particular embodiment the active agent of the present disclosure is sclareolide prepared by using *Salvia sclarea* flowers.

In another particular embodiment the active agent of the present disclosure is the sclareolide in solid form at room temperature and more preferably is sclareolide in the form of a white crystalline solid.

The present disclosure provides cosmetic and pharmaceutical formulations comprising the active agents (including the acceptable salts thereof), in physiologically acceptable carriers for oral and topical or transdermal administration.

Advantageously cosmetic or pharmaceutical formulations are in a form suitable for topical application and may take the form of, for example, a liquid, lotion, aerosol, cream, gel, ointment, rinse off formula or shampoo.

When the active agent is the sclareolide and the composition is in the form of a shampoo the solubility of the sclareolide can be enhanced by using an encapsulation system or a surfactant soluble solubilizer.

In one embodiment, wherein the active agent is the sclareolide, white crystals of sclareolide are encapsulated in naturally occurring cyclodextrin (α-CD, β-CD, HP-β-CD, and γ-CD) which greatly improves solubility and dissolution and facilitates the formulation of sclareolide into hair care compositions. The ratio of sclareolide to cyclodextrin could varies from 1:1 to 1:100, depending on the cyclodextrin types.

Encapsulation may also improve bioavailability, physicochemical stability, shelf life, reduce or eliminate unpleasant taste and smell, reduce irritation or sensitization, prevent active-active or active-other ingredients interactions and convert liquid actives into microcrystalline or amorphous powders.

Advantageously, encapsulated sclareolide can be used in the formulation of shampoos, conditioner, as well as hair serum.

In another particular embodiment, wherein the active agent is the sclareolide and the personal care composition is a surfactant-based system such as a shampoo, a surfactant soluble solubilizer C12-C15 Alkyl Lactate sold by Ashland under the trade name Ceraphyl 41 ester can advantageously be used to dissolve the sclarolide in a minimum ratio of 1:2.5 sclarolide/C12-C15 Alkyl Lactate.

In a particular embodiment the present disclosure relates to a method for improving scalp hydration, which comprises applying to the scalp a composition comprising an active agent selected among sclareol, sclareol-like, sclareolide or sclareolide-like and a physiologically acceptable carrier.

In a particular embodiment not covered by the claims, the present disclosure relates to a method for decreasing scalp sebum, which comprises applying to the scalp a composition comprising an active agent selected among sclareol, sclareol-like, sclareolide or sclareolide-like and a physiologically acceptable carrier.

In a particular embodiment not covered by the claims, the present disclosure relates to a method for improving scalp pH, which comprises applying to the scalp a composition comprising an active agent selected among sclareol, sclareol-like, sclareolide or sclareolide-like and a physiologically acceptable carrier.

In a particular embodiment the present disclosure relates to a method for decreasing scalp sensitivity (itching), which comprises applying to the scalp a composition comprising an active agent selected among sclareol, sclareol-like, sclareolide or sclareolide-like and a physiologically acceptable carrier.

In a particular embodiment not covered by the claims, the present disclosure relates to a method for reinforcing skin barrier function, which comprises applying to the scalp a composition comprising an active agent selected among sclareol, sclareol-like, sclareolide or sclareolide-like and a physiologically acceptable carrier.

Scalp skin biopsies cultures treated with a composition comprising 0.12% of sclareolide could increase keratin 10 expression and total lipid content, which both play a role in the reinforcement of skin barrier function.

In a particular embodiment the present disclosure relates to a method for eliminating or reducing adherent and loose dandruff which comprises applying to the scalp a composition comprising an active agent selected among sclareol, sclareol-like, sclareolide or sclareolide-like and a physiologically acceptable carrier.

In another particular embodiment the present disclosure relates to a method for decreasing the growth of *Malassezia* species which comprises applying to the scalp a composition comprising an active agent selected among sclareol, sclareol-like, sclareolide or sclareolide-like and a physiologically acceptable carrier.

Effects of active agents as antidandruff agents were studied on selected *in vitro* models develop to mimic both healthy commensal population versus disbalanced populations involved in scalp problems such as dandruff, itch, and dryness.

Clinical studies were also conducted to confirm the benefits of using active agents as in a scalp treatment that targets the root cause of dysbiosis and restores the equilibrium of the scalp microbiome.

In one embodiment the active agent is chosen among sclareol, sclareol-like, sclareolide or sclareolide-like in a concentration ranging from 0.001% to 2.0% by weight, relative to the total weight of the composition.

In another embodiment the active agent is the sclareolide in a rinse off formula in a concentration up to 0.5 %.

In another embodiment the active agent is chosen among sclareol, sclareol-like, sclareolide or sclareolide-like in a concentration ranging from 0.001% to 0.2% by weight, relative to the total weight of the composition.

In a preferred embodiment the active agent is sclareolide in a concentration ranging from 0.001% to 0.2% by weight, relative to the total weight of the composition.

In another preferred embodiment the active agent is sclareolide in a concentration of 0.12% by weight, relative to the total weight of the composition.

Results of the clinical study as presented in table 2 showed scalp care serum containing 0.12% sclareolide could significantly reduce adherent dandruff and loose dandruff weight reduce scalp sensitivity (itching) and improve skin barrier function; increase scalp hydration and scalp sebum at both day 14 and day 28 as compared to placebo, since dandruff was negatively associated with the sebum and water content.

Results of the anti-fungal efficacy of zinc pyrithione, piroctone, climbazole and sclareolide on *Malassezia furfur,* as presented at table 3, showed the germ count reduction % of scalp serum with 0.12% sclareolide as compared with placebo is similar or lower than the same amount of piroctone or climbazole added.

A composition comprising from 0.0018 to 0.12% of sclareolide showed antifungal properties on Malassezia species and more specifically on *Malassezia restricta,* in MIC (Minimum inhibitory concentration) assay (EUCAST guidelines, with slight modifications). In parallel, Zinc pyrithione has been tested at the same concentrations and presented a high inhibition of *Malassezia restricta.*

In another embodiment the present disclosure relates to a method for improving hair growth which comprises applying to the scalp a composition comprising as an active agent sclareol, sclareol-like, sclareolide or sclareolide-like and a physiologically acceptable carrier.

Scalp skin biopsies cultures treated with a composition comprising 0.12% of sclareolide have shown an increase of the expression of the enzyme CYP27B1 that synthetized the active form of vitamin D ((1,25(OH)2D3) that could stimulate hair growth at follicle level.

Specific embodiments of this cosmetic treatment method also result from the above description. Other advantages and features of the invention will be more apparent upon reading the examples provided for illustrative and non-limiting purposes.

### EXAMPLES

The following examples are intended to illustrate particular embodiments of the present invention but are by no means intended to limit the scope of the present invention.

### Example 1: To evaluate anti-dandruff effect and improvements of semi-healthy scalp skin condition after 4-week treatment using a scalp serum containing 0.12% sclareolide.

**Objective:** To evaluate anti-dandruff effect and improvements of semi-healthy scalp skin condition after 4-week treatment using a scalp serum containing 0.12% sclareolide (w/w).

The formula of the scalp serum is described below in table 1.

**Table 1**

| **Formulae** | **INCI (International Nomenclature of Cosmetic Ingredients) name** | **w/w (%)** | **Suppliers** |
|---|---|---|---|
| **Deionized water** | Deionized water | 70.79 | **Local** |
| **EDTA-2Na** | EDTA-2Na | 0.05 | **Sinopharm** |
| **2% Natrosol 250 GR (Water solution)** | Hydroxyethyl cellulose | 10.00 | **Ashland** |
| **1% Ashland 980 Carbomer (Water solution)** | Carbomer | 13.50 | **Ashland** |
| **Lubrajel Oil** | Glycerin (and) Glyceryl Acrylate/Acrylic Acid Copolymer (and) Propylene Glycol (and) PVM/MA Copolymer | 3.00 | **Ashland** |
| **SimulGel FL** | Hydroxyethyl Acrylate / Sodium Acryloyl dimethyl Taurate Copolymer & Isohexadecane & Polysorbate 60 | 0.10 | **SEPPIC** |
| **Sclareolide Kosher** | Sclareolide | 0.12 | **Ashland** |
| **Ceraphyl 230** | Diisopropyl Adipate | 1.00 | **Ashland** |
| **Prolipid 141** | Stearic acid, Behenyl alcohol, Glyceryl Stearate, Lecithin, Myristyl Lauryl Cetyl alcohol mixture, Palmitic acid | 0.50 | **Ashland** |
| **NaOH (10% water solution)** | Sodium hydroxide | 0.24 | **Sinopharm** |
| **Optiphen GP** | Phenoxyethanol, Proranediol, Propylene Carbonate, Caprylhydroxamic acid, o-Cymen-5-OL | 0.70 | **Ashland** |
| **Total** | | **100.00** | |

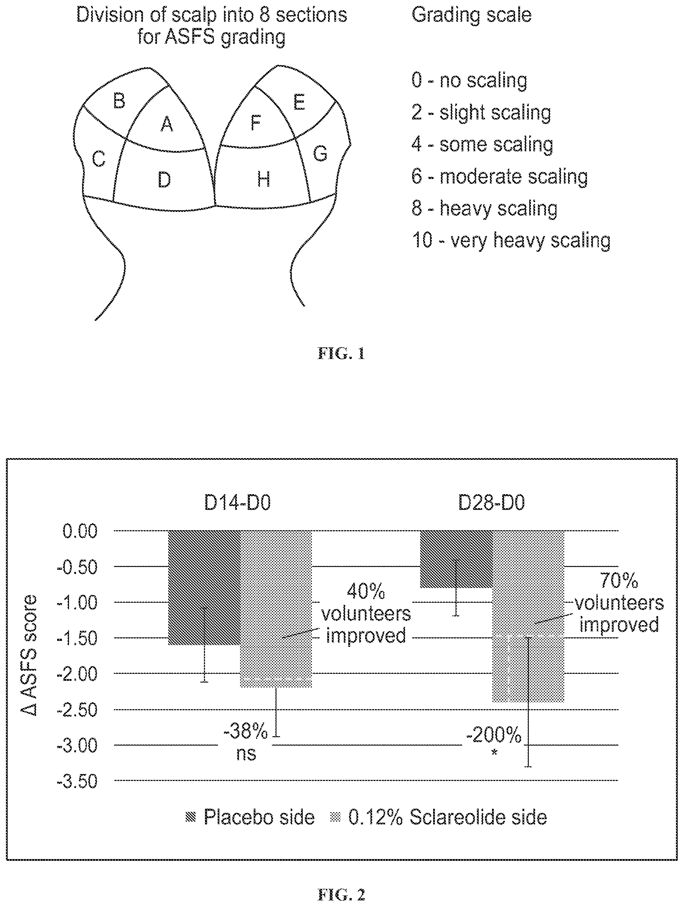

**Study design:** Double-blind, randomized, split-scalp, 4-week in use study
**Test Material:** Formula with/without 0.12% sclareolide
**Subject selection:** 10 subjects were used to evaluate anti-dandruff effect at test time point D0, D14, D28
**Inclusion criteria:**
   1) 18-65 years old.
   2) Subjects suffering from dandruff of scalp.
   3) Subjects who claims having a sensitive scalp skin (screened with 3S questionnaire).
   4) Patients who committed not to use medicated/non medicated shampoos/soaps (including soaps containing antibacterial/antifungal agents) or any other antidandruff treatment/hair products (including prescription and nonprescription medications such as hair oil, conditioners) for the entire duration of the study.
**Study evaluation:**
   Products applied on each half scalp (active versus placebo), measurements including:
   Expert grading on dandruff according to adherent scalp flaking score (ASFS), as presented in Fig. 1
   Self-evaluation of scalp sensitivity
   Loose dandruff weight
   Skin barrier function (Tewameter)
   Skin hydration (Dermalab)
   Skin sebum (Sebumeter)

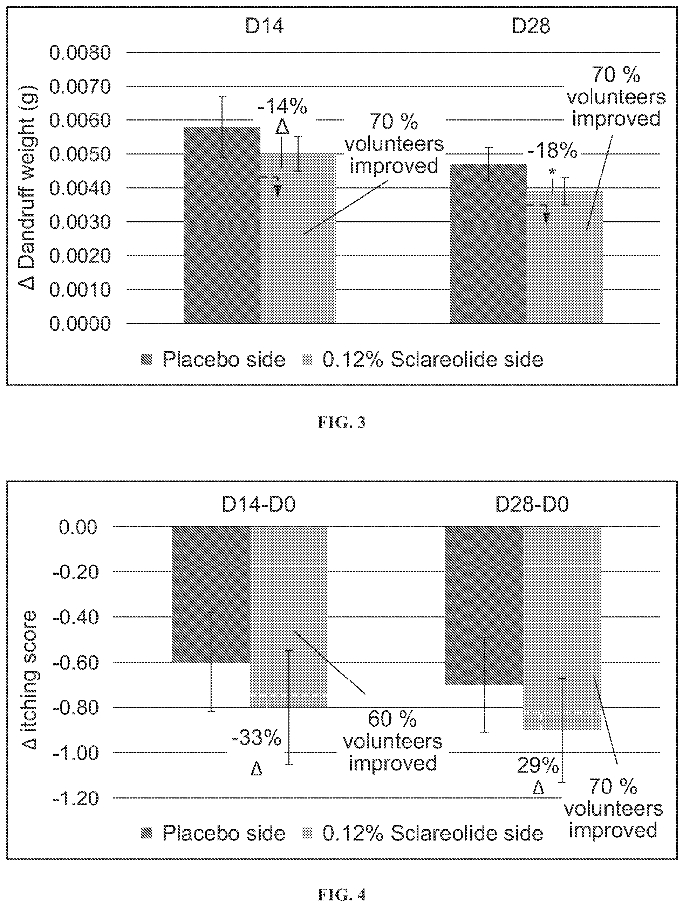

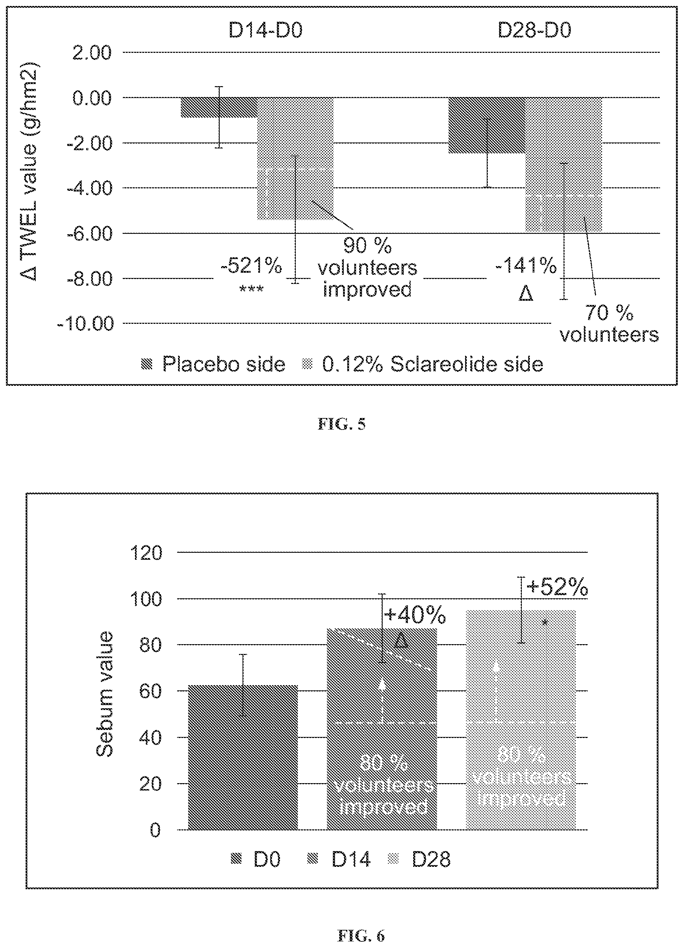

**Results are summarized in Table 2 and illustrated at** **Fig. 2 to Fig. 7**

**Table 2**

| | | |
|---|---|---|
| | | |
| ASFS (adherent dandruff) | D14**, D28* | (D14-D0)^{ns}, (D28-D0)* |
| Loose dandruff weight | D28**** (vs D14) | D14^{Δ}, D28* |
| Scalp sensitivity (itching) | D14**, D28*** | (D14-D0)^{Δ}, (D28-D0)^{Δ} |
| TEWL (Trans Epidermal Water Loss) | D14*, D28* | (D14-D0)***, (D28-D0)^{Δ} |
| Scalp pH | D14***, D28**** | D14^{Δ}, D28^{Δ} |
| Scalp hydration | D14^{Δ}, D28* | ns |
| Scalp sebum | D14^{Δ}, D28* | ns |
| Image show | Demoscopy imaging (CBS) | |
| | Scalp erythema imaging (Tivi-700) | |
| | Desquamation Index (tape + Visioline VL650) | |

**Results and conclusion:** The clinical study showed scalp serum containing 0.12% sclareolide could significantly reduce adherent dandruff and loose dandruff weight reduce scalp sensitivity (itching) and improve skin barrier function; increase scalp hydration and scalp sebum at both day 14 and day 28 as compared to placebo, since dandruff was negatively associated with the sebum and water content.

### Example 2: Evaluation of sclareolide antifungal potential on Malassezia restricta

Sclareolide antifungal potential on *Malassezia restricta* was evaluated by the broth microdilution method.

**Method:** *Malassezia restricta* was routinely grown on modified Leeman-Notman (mLN) agar. The broth microdilution assay was performed following EUCAST guidelines (www.eucast.org) with slight modifications. Between five and ten colonies were suspended in a modified RPMI 1640 media supplemented with resazurin at a D.O (Density Optical) of 0.1. In a 96 well plate, serial dilutions of sclareolide (0.00023% to 0.12%), zinc pyrithione (0.015% - 0.0023%) and Ketoconazol (0.5 - 0.015 µg/ml) were prepared in modified RPMI 1640. An equal volume of *Malassezia restricta* inoculum was added to the test samples at different concentrations. Following 48h-60h of incubation at 34°C, resazurin fluorescence was analyzed at 530 nm and 590 nm.

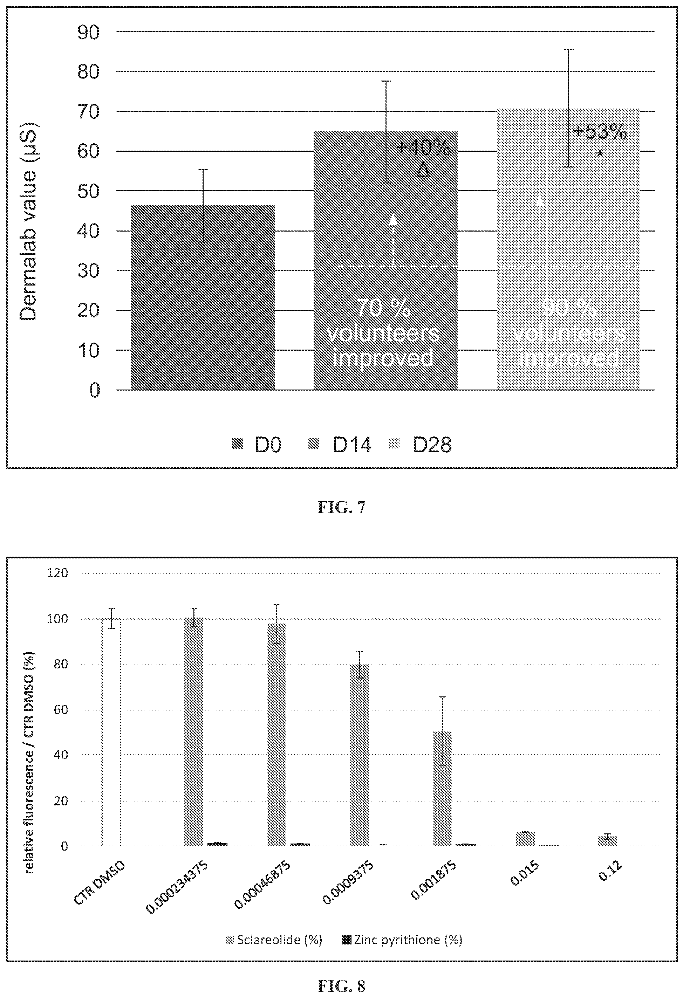

**Results** are presented as relative fluorescence intensity compared to DMSO control. the MIC is defined as the lowest drug concentration that inhibited growth.
Growth *of Malassezia restricta* was significantly inhibited by sclareolide from the concentration 0.001875%, and completely inhibited at the concentration 0.12%, as illustrated at Fig. 8.

### Example 3 Anti-fungal efficacy of Zinc Pyrithione, Piroctone, Climbazole and sclareolide on

### Malassezia furfur

Assay of the anti-fungal efficacy of zinc pyrithione, piroctone, climbazole and sclareolide on *Malassezia furfur* was assayed. Zinc pyrithione, piroctone and climbazole do not form part of the claimed invention.

Results showed the germ count reduction (%) of scalp serum with 0.12% sclareolide (as described in example 1) when compared to placebo is similar or lower than the same amount of piroctone or climbazole added as presented at table 3.

**Table 3**

| | 0 12%OCT | 0 12% ZPT | 0 12 Climbazole | 0 12%sclateolide |
|---|---|---|---|---|
| Reduction % (6h) | 15.4% | 47.4% | 38.5% | 38.5% |
| Reduction % (24h) | 35.3% | 93.1% | 33.8% | 32.4% |

### Example 4: Evaluation of the effect of sclareolide on CYP27b1 expression in ex vivo scalp skin biopsies:

The aim of this study is to show the effect of sclareolide on the expression of CYP27B 1 in biopsies of healthy scalp skin.

**Method:** CYP27B1 was assessed by immunohistochemistry with a specific antibody. Biopsies of healthy human scalp skin in culture were treated with sclareolide diluted at 0.12% in placebo (ceraphyl 368 (Ashland) applied once a day for 48 hours topically (20 µl/biopsy). Control biopsies received only ceraphyl 368. The immunostaining was performed using paraffin sections incubated in the presence of anti-CYP27B 1 antibody (rabbit monoclonal, Abcam). After an hour and a half of incubation followed by rinses, the sections were incubated in the presence of the secondary anti-rabbit antibody coupled with a fluorophore (Alexa Fluor^{®} 488, Invitrogen). The sections were then examined under a fluorescence microscope (Nikon Eclipse Ni-E microscope). CYP27B1 expression was then observed (using NiS-AR Nikon acquisition software) and quantified by image analysis (Volocity^{®} image analysis software, Improvision).

**Results** are presented in table 4. Treatment with 0.12% sclareolide showed an increase in CYP27B1 labelling intensity in scalp skin biopsies after 48-hour treatment (+38%, very significant compared to biopsies treated with the placebo).

**Table 4**

| Item | placebo | 0.12% Sclareolide |
|---|---|---|
| CYP27B 1 protein expression (% placebo) | 100% | 138% |

**Conclusion:** Sclareolide at 0.12% was able to increase the level of CYP27B1 in healthy *ex vivo* scalp skin biopsies. This effect may be associated with a higher quantity of active form of vitamin D in scalp that could stimulates hair growth at follicle level.

### Example 5: Evaluation of the effect of sclareolide on keratin 10 expression in ex vivo scalp skin biopsies:

The aim of this study is to show the effect of sclareolide on the expression of keratin 10 in biopsies of healthy scalp skin.

**Method:** Keratin 10 was assessed by immunohistochemistry. The protocol was the same as in example 4 with the use of anti-keratin 10 antibody (rabbit monoclonal, Abcam).

**Results** are presented in table 5. Treatment with 0.12% sclareolide showed an increase in keratin 10 labelling intensity in scalp skin biopsies after 48-hour treatment (+20%, highly significant compared to biopsies treated with the placebo).

**Table 5**

| Item | placebo | 0.12% Sclareolide |
|---|---|---|
| Keratin 10 expression (% placebo) | 100% | 120% |

**Conclusion:** Sclareolide at 0.12% was able to increase the level of keratin 10 in healthy *ex vivo* scalp skin biopsies. This effect may be associated with a better quality of the skin barrier function in the scalp.

### Example 6: Evaluation of the effect of sclareolide on total lipid content in ex vivo scalp skin biopsies:

The aim of this study is to show the effect of sclareolide on the synthesis of lipids in biopsies of healthy scalp skin.

**Method:** total lipids were assessed by fluorescent staining by using the fluorescent dye Nile Red. The treatment of the biopsies was the same than in example 4. The staining was performed using paraffin sections incubated with 100nM of Nile Red solution (Sigma) for 10 minutes. The sections were then examined under a fluorescence microscope (Nikon Eclipse Ni-E microscope). Red fluorescence was then observed (using NiS-AR Nikon acquisition software) and quantified by image analysis (Volocity^{®} image analysis software, Improvision).

**Results** are presented in table 6. Treatment with 0.12% sclareolide showed an increase in lipid labelling intensity in scalp skin biopsies after 48-hour treatment (+46%, significant compared to biopsies treated with the placebo).

**Table 6**

| Item | placebo | 0.12% sclareolide |
|---|---|---|
| Total lipid content (% placebo) | 100% | 146% |

**Conclusion:** Sclareolide at 0.12% was able to increase the level of lipids in healthy ex *vivo* scalp skin biopsies. This effect may be associated with a better quality of the skin barrier function in the scalp.

### Example 7: Encapsulation of sclareolide

Sclareolide is white crystals with limited solubility in water and oils, which will be difficult to use in personal care application. To address this issue, sclareolide encapsulated by cyclodextrin (CD) was proposed. The surface of the cyclodextrin molecules makes them water soluble, but the hydrophobic cavity provides a microenvironment for appropriately sized nonpolar molecules, such as sclareolide. The naturally occurring CDs are α-CD, β-CD, HP- β-CD, and γ-CD synthesized by fermentation or enzymatically. Inclusion complexes formed with a host-guest molecule may exhibit improved chemical or biological properties compared to the host molecule alone. Such inclusion may improve solubility, dissolution, and bioavailability; increase the physicochemical stability of actives and improve the shelf life of actives; reduce or eliminate unpleasant taste and smell; reduce irritation or sensitization; prevent active-active or active-other ingredients interactions; convert liquid actives into microcrystalline or amorphous powders, etc.

Cyclodextrins are commercially available for example from WACKER, such as CAVAMAX^{®} W6 (α-cyclodextrin), CAVAMAX^{®} W7 (β-cyclodextrin), CAVAMAX^{®} W8 (γ-cyclodextrin), CAVASOL^{®} W6 HP (hydroxypropyl-α-cyclodextrin), CAVASOL^{®} W7 HP (hydroxypropyl-β-cyclodextrin), CAVASOL^{®} W8 HP (hydroxypropyl-γ-cyclodextrin), CAVASOL^{®} W7 M (methylated-β-cyclodextrin).

White crystals of sclareolide could be encapsulated in in naturally occurring cyclodextrin (α-CD, β-CD, HP-β-CD, and γ-CD) with the ratio of sclareolide to cyclodextrin from 1:1 to 1:100, depending on the cyclodextrin types. As showed in Fig.1, sclareolide could not be dissolved in 12% Sodium laureth sulfate (SLES)/2% Cocamidopropyl betaine (CAPB) after 1 hour stirring, while HP- β-cyclodextrin encapsulated sclareolide (sclareolide to cyclodextrin ratio 1:6) could be dissolved within 4-5 mins.

Fig.9 shows the solubility of Sclareolide complex in surfactant solution after 1 hour (12% Sodium laureth sulfate /2% Cocamidopropyl betaine)

Fig.10 shows the differential Scanning Calorimeter (DSC) curve of sclareolide and Sclareolide complex 1 (HP- β-cyclodextrin) and 2 (γ- cyclodextrin). The crystallite peak of sclareolide appears around 127.00 °C, while no peak appears in the curves of both sclareolide complexes which means sclareolide has been encapsulated in cyclodextrin completely. +k+

## Claims

1. A composition comprising sclareolide as an active agent and a physiologically acceptable carrier, for its therapeutic use in improving the scalp condition and hair growth, wherein improving the scalp condition is chosen among at least one of decreasing scalp sensitivity (itching), eliminating or reducing dandruff and decreasing the growth of Malassezia species, and wherein the composition comprises sclareolide in a concentration ranging from 0.001% to 0.2% by weight, relative to the total weight of the composition.

2. The composition for use according to claim 1 wherein Malassezia species is *Malassezia restricta* or *Malassezia furfur.*

3. The composition for use according to claim 1, wherein the composition is applied for at least 14 days.

4. The composition for use according to claim 1 for use in improving hair growth, wherein the compositior comprises 0.12 % of sclareolide.

5. The composition for use according to claim 1, wherein the level of the enzyme CYP27B1 is increased.

6. The composition for use according to claim 1, wherein keratin 10 expression and total lipid content are increased.

7. The composition for use according to claim 1, wherein the composition is suitable for topical application,

8. The composition for use according to claim 1, wherein the composition is in the form of a liquid, a lotion, an aerosol, a cream, a gel, an ointment, a rinse off formula or a shampoo.

9. The composition for use according to claim 1, wherein the composition is in the form of a shampoo anc wherein the solubility of the sclareolide is enhanced by using an encapsulation system.

10. Cosmetic use of a composition comprising sclareolide as an active agent and a physiologically acceptable carrier, for improving the scalp condition wherein improving the scalp condition is improvement of scalp hydration and wherein the composition comprises sclareolide in a concentration ranging from 0.001% to 0.2% by weight, relative to the total weight of the composition.

## Patentansprüche

1. Eine Zusammensetzung umfassend Sclareolid als Wirkstoff und einen physiologisch verträglichen Träger zur therapeutischen Verwendung zur Verbesserung des Zustands der Kopfhaut und des Haarwachstums, wobei die Verbesserung des Zustands der Kopfhaut aus mindestens einem der folgenden Punkte ausgewählt ist: Verringerung der Empfindlichkeit der Kopfhaut (Juckreiz), Beseitigung oder Verringerung von Schuppen und Verringerung des Wachstums von Malassezia-Arten, und wobei die Zusammensetzung Sclareolid in einer Konzentration im Bereich von 0,001 bis 0,2 Gew.-%, bezogen auf das Gesamtgewicht der Zusammensetzung, enthält.

2. Die Zusammensetzung zur Verwendung gemäß Anspruch 1, wobei es sich bei den Malassezia-Arten um *Malassezia restricta* oder *Malassezia furfur* handelt.

3. Die Zusammensetzung zur Verwendung gemäß Anspruch 1, wobei die Zusammensetzung mindestens 14 Tage lang angewendet wird.

4. Die Zusammensetzung zur Verwendung gemäß Anspruch 1 zur Verbesserung des Haarwachstums, wobei die Zusammensetzung 0,12 % Sclareolid enthält.

5. Die Zusammensetzung zur Verwendung gemäß Anspruch 1, wobei der Spiegel des Enzyms CYP27B1 erhöht wird.

6. Die Zusammensetzung zur Verwendung gemäß Anspruch 1, wobei die Keratin-10-Expression und der Gesamtlipidgehalt erhöht werden.

7. Die Zusammensetzung zur Verwendung gemäß Anspruch 1, wobei die Zusammensetzung zur topischen Anwendung geeignet ist.

8. Die Zusammensetzung zur Verwendung gemäß Anspruch 1, wobei die Zusammensetzung in Form einer Flüssigkeit, einer Lotion, eines Aerosols, einer Creme, eines Gels, einer Salbe, einer abwaschbaren Formulierung oder eines Shampoos vorliegt.

9. Die Zusammensetzung zur Verwendung gemäß Anspruch 1, wobei die Zusammensetzung in Form eines Shampoos vorliegt und wobei die Löslichkeit des Sclareolids durch die Verwendung eines Verkapselungssystems verbessert wird.

10. Eine kosmetische Verwendung einer Zusammensetzung, die Sclareolid als Wirkstoff und einen physiologisch verträglichen Träger umfasst, zur Verbesserung des Zustands der Kopfhaut, wobei die Verbesserung des Zustands der Kopfhaut eine Verbesserung der Feuchtigkeitsversorgung der Kopfhaut ist und wobei die Zusammensetzung Sclareolid in einer Konzentration im Bereich von 0,001 bis 0,2 Gew.-%, bezogen auf das Gesamtgewicht der Zusammensetzung, umfasst.

## Revendications

1. Composition comprenant du sclaréolide en tant qu'agent actif et un support physiologiquement acceptable, destinée à son utilisation thérapeutique pour améliorer l'état du cuir chevelu et la croissance des cheveux, dans laquelle l'amélioration de l'état du cuir chevelu est choisie parmi au moins l'une des actions suivantes: diminuer la sensibilité du cuir chevelu (démangeaisons), éliminer ou réduire les pellicules et diminuer la prolifération des espèces de Malassezia, et dans laquelle la composition comprend du sclaréolide à une concentration comprise entre 0,001 % et 0,2 % en poids, par rapport au poids total de la composition.

2. La composition destinée à l'usage selon la revendication 1, dans laquelle l'espèce de Malassezia est *Malassezia restricta* ou *Malassezia furfur.*

3. La composition destinée à l'usage selon la revendication 1, dans laquelle la composition est appliquée pendant au moins 14 jours.

4. La composition destinée à l'usage selon la revendication 1 pour améliorer la croissance des cheveux, dans laquelle la composition comprend 0,12 % de sclaréolide.

5. La composition destinée à l'usage selon la revendication 1, dans laquelle le taux de l'enzyme CYP27B1 est augmenté.

6. La composition destinée à l'usage selon la revendication 1, dans laquelle l'expression de la kératine 10 et la teneur totale en lipides sont augmentés.

7. La composition destinée à l'usage selon la revendication 1, dans laquelle la composition est adaptée à une application topique.

8. La composition destinée à l'usage selon la revendication 1, dans laquelle la composition se présente sous la forme d'un liquide, d'une lotion, d'un aérosol, d'une crème, d'un gel, d'une pommade, d'une formule à rincer ou d'un shampoing.

9. La composition destinée à l'usage selon la revendication 1, dans laquelle la composition se présente sous la forme d'un shampoing et dans laquelle la solubilité du sclaréolide est améliorée par l'utilisation d'un système d'encapsulation.

10. Utilisation cosmétique d'une composition comprenant du sclaréolide en tant qu'agent actif et un support physiologiquement acceptable, pour améliorer l'état du cuir chevelu, dans laquelle l'amélioration de l'état du cuir chevelu consiste en une amélioration de l'hydratation du cuir chevelu et dans laquelle la composition comprend du sclaréolide à une concentration comprise entre 0,001 % et 0,2 % en poids, par rapport au poids total de la composition.
